# EUROPÄISCHE PATENTSCHRIFT

(11) **EP 1 132 062 B1**
(45) Veröffentlichungstag und Bekanntmachung des Hinweises auf die Patenterteilung: **04.08.2004**
(21) Anmeldenummer: 01810125.3
(22) Anmeldetag: 07.02.2001
(51) Int. Cl.: A61F 2/30

(54) **Einzementierbare Schaftprothese**
Cement affixated shaft prosthesis
Prothèse de tige à fixation par ciment

(30) Priorität: 01.03.2000 EP 00810171
(43) Veröffentlichungstag der Anmeldung: 12.09.2001
(73) Patentinhaber: Sulzer Orthopedics Ltd., 6340 Baar (CH)
(72) Erfinder: Güttinger, Werner, 8465 Rudolfingen (CH); Mettler, Felix, 8409 Winterthur (CH)
(74) Vertreter: Manitz, Finsterwald & Partner Gbr

(56) Entgegenhaltungen:
- EP-A- 0 483 057
- DE-A- 19 613 081
- GB-A- 2 104 391
- US-A- 5 425 768

## Beschreibung

Die Erfindung handelt von einer einzementierbaren Schaftprothese mit einer an ihrem distalen Schaftende aufsteckbaren Zentriervorrichtung aus Kunststoff, welche zum distalen Schaftende einen Hohlraum bildet, wobei zwischen dem distalen Schaftende und der Zentriervorrichtung eine konische Klemmverbindung besteht, die in distaler Richtung ein tieferes Einsinken des distalen Schaftendes in den Hohlraum ermöglicht.

In der Patentanmeldung GB-A-2 104 391 wird eine Zentriervorrichtung für das distale Ende eines einzementierbaren Prothesenschaftes gezeigt, die auf das distale Ende aufsteckbar ist und einen Hohlraum für das distale Ende bildet, damit das distale Ende zur Vermeidung von Rissen im ausgehärteten Zement tiefer in den Hohlraum einsinken kann. Als Werkstoff werden Kunststoffe wie Polyethylen für die Zentriervorrichtung vorgesehen.

Diese vorgeschlagene Ausführung hat den Nachteil, dass das distale Ende, welches konisch ausgebildet ist in sehr engen Toleranzen in seiner Aussenkontur bearbeitet werden muss, um in axialer Richtung ein Klemmen und bei wachsender Axialkraft ein Nachrutschen zu gewährleisten. Diese Art der Klemmung verursacht gleichzeitig eine dermassen grosse Dichtwirkung, dass eine Entlüftungsbohrung am tiefsten Punkt des Hohlraumes vorgeschlagen wird. Ein weiterer Nachteil besteht darin, dass dicke Schaftenden, die zur Einhaltung eines gleichmässig dünnen Zementmantels in der Markraumhöhle erwünscht wären, im distalen Bereich grosse Oberflächenanteile mit einem relativ grossen halben Konuswinkel der Oberfläche zur Schaftachse aufweisen. Wenn in diesem Bereich eine Klemmung mit eng anliegenden Führungsflächen vorgesehen ist, treten wegen der relativ grossen halben Konuswinkel nach dem Aushärten des Knochenzements grosse Axialkräfte und Ringspannungen an einem den Schaft und die Zentriervorrichtung umschliessenden Zementköcher auf, falls der Schaft weiter in den Hohlraum einsinkt.

Es ist Aufgabe der Erfindung Prothese und Zentriervorrichtung in dieser Hinsicht zu verbessern. Dies wird entsprechend dem unabhängigen Anspruch 1 dadurch erreicht, dass das distale Schaftende in proximaler Richtung eine konische Bohrung aufweist, in der ein im Hohlraum an die Zentriervorrichtung angeformter Zapfen die Klemmverbindung bildet.

Die Erfindung hat den Vorteil, dass die Aussenkontur des distalen Prothesenendes keine Führungsfunktion mehr hat und dass der Hohlraum der Zentriervorrichtung durch eine schwache Linienberührung zwischen deren proximal umlaufender Kante und der Oberfläche des Schaftes zementfrei gehalten werden kann. Ein weiterer Vorteil besteht in der Sicherheit der Klemmung und Positionierung der Zentriervorrichtung auf dem Zapfen, da der Konuswinkel frei wählbar ist und da eine konische Bohrung im distalen Schaftende kostengünstig in engen Toleranzen herstellbar ist.

Vorteilhafte Weiterbildungen der Erfindung ergeben sich aus den abhängigen Ansprüchen 2 bis 10.

Bei der Herstellung der Zentriervorrichtung in einem Spritzwerkzeug ergeben sich besonders enge Paarungstoleranzen im Klemmbereich zwischen Zapfen und konischer Bohrung, da die Geometrie des Zapfens wegen der Formumkehr in der Spritzform ebenfalls als Bohrung erzeugt wird. Herstellung und die Kontrolle der Toleranzen sind so an beiden Teilen, Schaftende und Zentriervorrichtung, einfacher.

Der halbe Konuswinkel der konischen Bohrung kann je nach Material von Schaft und Zentriervorrichtung und nach Geometrie des Zapfens zwischen 0,5° und 2,5° gewählt werden. Bei einem metallischen Schaft und einer Zentriervorrichtung aus Kunststoff beispielsweise aus PMMA (Polymethylmetacrylat) kann der halbe Konuswinkel zwischen 1,2° und 1,6° liegen.

Wenn der Zapfen zwei Stufen mit unterschiedlichem Durchmesser aufweist, und die Stufung zum axialen Abstand so gewählt ist, dass sie mit dem halben Konuswinkel der konischen Bohrung zusammenfällt, dann entstehen beim Aufstecken der Zentriervorrichtung zwei axial beabstandete Führungs- und Klemmzonen, die entsprechend der Reibung und dem halben Konuswinkel zwischen konischer Bohrung und Zapfen in axialer Richtung eine Widerstandskraft F entgegensetzen, welche mit fortschreitendem Eintauchen der Schaftspitze überproportional wächst. Ein Eintauchen ist praktisch nur deswegen möglich, weil sich die Stufen des Zapfens radial nach innen deformieren. Wenn der Abstand der Stufen so gewählt wird, dass zunächst nur eine Stufe an der konischen Bohrung ansteht und wenn diese Stufe, weil sie zum Beispiel nur aus wenigen Rippen gebildet wird, sich radial leichter deformieren lässt, dann entsteht beim Aufstecken der Zentriervorrichtung zunächst nur eine geringe axiale Widerstandskraft, die erst viel stärker anwächst, wenn auch die zweite Stufe beim weiteren Eintauchen der distalen Schaftspitze radial nach innen deformiert wird. Das heisst beim Aufstecken hält sich die Zentriervorrichtung von selbst auf der Schaftspitze und bildet einen spürbaren Widerstand, wenn die Zentriervorrichtung ausgerichtet mit ihrem Zapfen eine vorgesehene Position erreicht hat. Der Prothesenschaft lässt sich anschliessend mit der Zentriervorrichtung in einen mit Knochenzement gefüllten Markraum eines Röhrenknochens einführen und wird an seinem distalen Ende durch mindestens drei Flossen der Zentriervorrichtung geführt.

Die Sollposition der Zentriervorrichtung wird noch markanter beim Aufstecken signalisiert, wenn die konische Bohrung einen Einlaufkonus mit etwas grösserem halben Konuswinkel aufweist und die zweite Stufe an diesem steileren Konus ansteht und zum weiteren Eindringen in die konische Bohrung zunächst an diesem steileren Konus radial deformiert werden muss. Der Hohlraum in der Zentriervorrichtung und die axiale Widerstandskraft sind so bemessen, dass das distale Schaftende nach dem Aufstecken und Einzementieren später mehrere Millimeter in die Zentriervorrichtung eintauchen kann, ohne dass der von Knochenzement eingeschlossene Mantel der Zentriervorrichtung unzulässige Kräfte in axialer Richtung erfährt.

Bei einer Femurschaftprothese ist es wegen des medial gelegenen Femurkopfes von Vorteil, wenn die Zentriervorrichtung beim Einführen des Schaftes in der Markraumhöhle diesen mit einer Flosse gegen lateral abstützt. Aus diesem Grund ist es weiterhin von Vorteil, wenn zwischen Schaftspitze und Zentriervorrichtung im Hohlraum eine von der Eintauchtiefe unabhängige Drehsicherung angebracht ist, die nur dann ein Aufstecken der Zentriervorrichtung gestattet, wenn eine Flosse gegen lateral ausgerichtet ist. Wenn die vorgefertigte Zentriervorrichtung selbst aus PMMA besteht, ergibt sich nach dem Einzementieren der Prothese ein homogener Zementköcher mit dem Knochenzement, der ein späteres Einsinken des Schaftes zulässt, ohne dass im Bereich der distalen Schaftspitze unzulässige Spannungen im Zementköcher entstehen.

Im folgenden wird die Erfindung anhand von Ausführungsbeispielen beschrieben. Es zeigen:
- Fig. 1: Schematisch eine Ansicht auf eine Femurschaftprothese mit einer Zentriervorrichtung;
- Fig. 2: schematisch einen vergrösserten Ausschnitt des Schaftendes von Figur 1 mit der Zentriervorrichtung;
- Fig. 3: schematisch eine Draufsicht auf die Zentriervorrichtung von Figur 2;
- Fig. 4: schematisch, stark vergrössert und in den Winkeln überhöht einen Ausschnitt von einem Zapfen beim Beginn des Aufliegens in einer konischen Bohrung;
- Fig. 5: schematisch die Anordnung von Figur 4 beim Beginn des Aufliegens einer zweiten Stufe in der konischen Bohrung;
- Fig. 6: schematisch die Anordnung von Figur 5, beim weiteren Einfahren des Zapfens;
- Fig. 7: schematisch eine weitere Anordnung wie in Figur 2, bei der die zweite Stufe zunächst an einem Einlaufkonus aufliegt;
- Fig. 8: schematisch eine Grafik für die Widerstandskraft in axialer Richtung beim Einpressen eines Zapfens; und
- Fig. 9: schematisch eine Anordnung analog zu Figur 5, bei der die zweite Stufe mit einem Punkt P₃ als Erste in der konischen Bohrung aufliegt.

Die Figuren zeigen eine einzementierbare Schaftprothese 1 mit einer an ihrem distalen Schaftende 3 aufsteckbaren Zentriervorrichtung 2 aus Kunststoff, welche zum distalen Schaftende 3 einen Hohlraum 4 bildet. Zwischen dem distalen Schaftende 3 und der Zentriervorrichtung 2 besteht eine Klemmverbindung, die in distaler Richtung ein tieferes Einsinken des Schaftendes in den Hohlraum ermöglicht. Die Klemmverbindung wird durch einen im Hohlraum 4 an die Zentriervorrichtung 2 angeformten Zapfen 7 erreicht, welcher in proximaler Richtung in eine konische Bohrung 5 des Schaftendes 3 hineinragt.

In den Figuren sind gleiche Hinweiszeichen für gleiche Funktionsmerkmale verwendet.

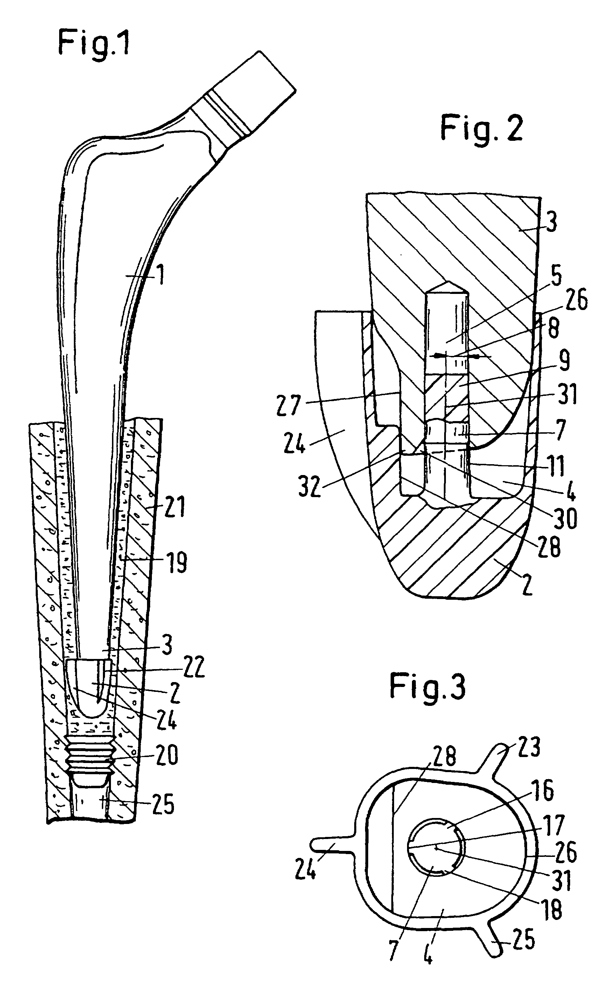

In Figur 1 ist eine Femurschaftprothese 1 an ihrem distalen Schaftende 3 mit einer aufsteckbaren Zentriervorrichtung 2 versehen. Der Femurknochen 21 ist in seiner Markraumhöhe 25 mit einer Markraumsperre 20 versehen, welche gegen flüssig eingefüllten Knochenzement 19 dichtet. Prothese 1 und Zentriervorrichtung werden gemeinsam in den flüssigen Knochenzement 19 abgesenkt bis eine vorgesehene Eindringtiefe erreicht ist. Während dem Absenken wird das distale Schaftende 3 durch Flossen 22, 24 innerhalb der Markraumhöhle 25 zentriert. Dabei ist eine Flosse 24 gegen lateral ausgerichtet.

Die Zentriervorrichtung 2 in Figur 2 und 3 hat mit ihrem oberen Rand 26 nur noch eine schwache Linienberührung zum Schaftende 3, um ein Eindringen von flüssigem Knochenzement zu verhindern. Die eigentliche Fixierung geschieht durch einen in einem Hohlraum 4 an der Zentriervorrichtung 2 angeformten Zapfen 7, der in einer konischen, in proximaler Richtung am Schaftende 3 angebrachten Bohrung 5 geklemmt ist. Für einen Metallschaft ist beispielsweise eine konische Bohrung 5 mit einem halben Konuswinkel 8 von 1,43° vorgesehen. Der Zapfen besitzt eine erste Stufe mit aufgesetzten Rippen 16, 17, 18, die im unverspannten Zustand auf einen Durchmesser 10 begrenzt sind, der ein wenig unter dem Durchmesser 12 einer anschliessenden zweiten Stufe 11 liegt. Die Stellung des Zapfens 7 in Figur 2 entspricht einer gewollten Aufsteckposition, in der die Rippen 16, 17, 18 bereits soweit radial nach innen deformiert sind, dass eine gewünschte Klemmkraft gegen Herausziehen erreicht ist. Gleichzeitig ist die zweite Stufe 11 mit ihrem geringfügig grösseren Durchmesser in einem Einlaufkonus 30 der konischen Bohrung 5 zentriert und bildet einen zusätzlichen und gut spürbaren Widerstand gegen das weitere Eindringen des Zapfens 7. Erst bei einer wesentlich grösseren Axialkraft des Schaftendes 3 wird auch die zweite Stufe 11 radial nach innen deformiert und das Schaftende 3 kann gegen eine vorgesehene Widerstandskraft tiefer in den Hohlraum 4 der Zentriervorrichtung 2 eintauchen. Der Hohlraum 4 und die konische Bohrung 5 sind so bemessen, dass der Schaft nach dem Aufstecken der Zentriervorrichtung 2 gegen einen höheren Widerstand noch mehrere Millimeter in die Zentriervorrichtung 2 eintauchen kann. Dieser "Sprung" im Widerstand hat den Vorteil, dass sich die Zentriervorrichtung 2 während dem Führen und Absenken des Prothesenschaftes 1 im flüssigen Knochenzement 19 nicht weiter auf den Schaft aufschiebt. Um die Zentriervorrichtung nur dann aufsetzen zu können, wenn eine Flosse 24 gegen lateral steht, ist eine abgesetzte Fläche 27 parallel zur Schaftachse 31 am Schaftende 3 angebracht, die in einer Nase 32 endet. Die Zentriervorrichtung besitzt dazu eine passende Führungsfläche 28 im Hohlraum 4, die eine Verdrehung verhindert.

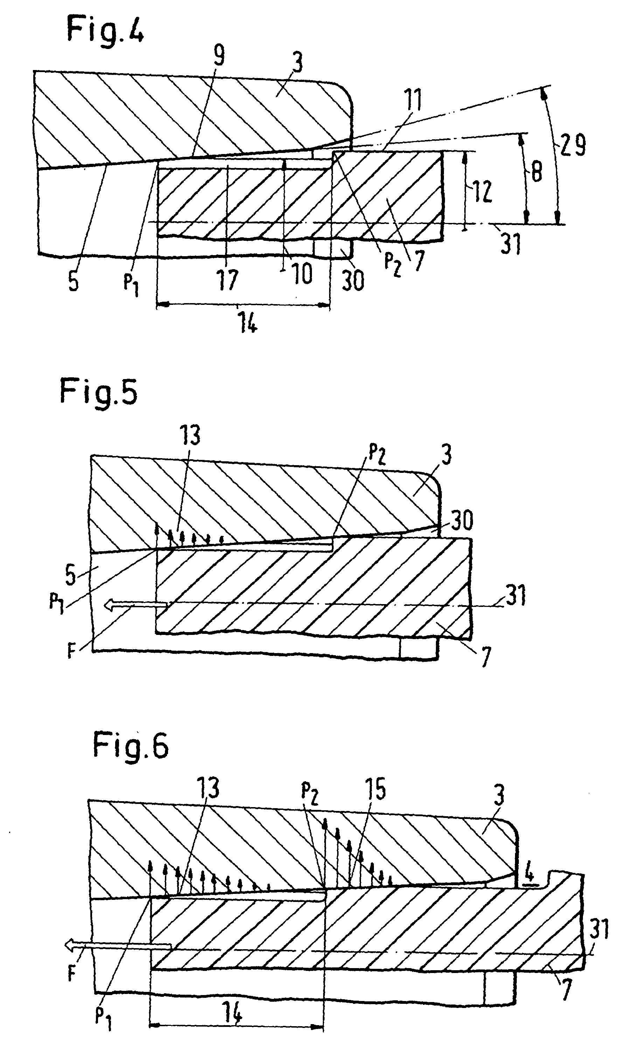

In den Figuren 4, 5, 6 ist eine Anordnung gezeigt, bei der der Einlaufkonus 30 mit seinem halben Konuswinkel 29 nicht zur Vergrösserung der Widerstandskraft F in axialer Richtung beiträgt. Beim Aufstecken des Zapfens 7 berührt zunächst die erste Stufe 9 mit einem Punkt P₁ auf Durchmesser 10 der Rippe 17 die konische Bohrung 5, während der axiale Abstand 14 für einen Punkt P₂ mit Durchmesser 12 der zweiten Stufe 11 so bemessen ist, dass der Punkt P₂ die konische Bohrung 5 noch nicht berührt. Bei der Fortsetzung der Aufsteckbewegung werden die Rippen 17 im Punkt P₁ radial nach innen deformiert und es entsteht eine Klemm- und Führungszone 13 (Fig. 5) mit einer schwach wachsenden Klemmkraft. Erst wenn die zweite Stufe 11, welche einen wesentlich grösseren Widerstand entgegensetzt, an der konischen Bohrung ansteht, ist eine Sollposition für das Aufstecken erreicht. Die Widerstandskraft in axialer Richtung ist so gross, dass sich Schaft 1 und Zentriervorrichtung während dem Absenken des Schaftes und seiner Führung durch die Zentriervorrichtung nicht weiter zusammenschieben. In Figur 6 hat sich der Schaft 3 gegen eine grössere Widerstandskraft F in den Hohlraum 4 hineinbewegt, wobei die Widerstandskraft F so bemessen ist, dass keine unzulässigen Zugspannungen in axialer Richtung von der Zentriervorrichtung 2 an dem sie umgebenden Zementköcher 19 erzeugt werden.

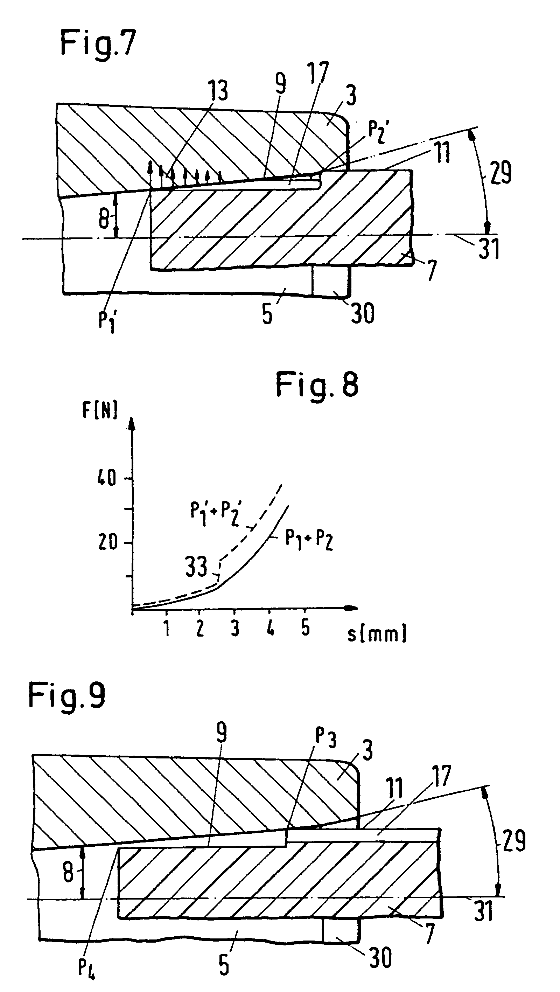

In Figur 7 ist analog zu Figur 5 der Zustand am Ende des Aufsteckens der Zentriervorrichtung gezeigt. Der Zapfen 7 ist bereits an seiner ersten Stufe 9 in Punkten P₁' deformiert worden und bildet Klemm- und Führungszonen 13, während Punkte P₂' der zweiten Stufe 11 an dem Einlaufkonus 30 mit halbem Konuswinkel 29 anliegen. In dem kurzen steileren Konusabschnitt, der von dem Punkt P₂' durchfahren werden muss, bis er auf der konischen Bohrung 5 mit halbem Konuswinkel 8 aufliegt, findet ein Sprung 33 der Widerstandskraft F statt. In diesem Bereich liegt auch eine sichere axiale Positionierung beim Aufstecken der Zentriervorrichtung.

Figur 8 zeigt eine Grafik in der die Widerstandkraft F über einem Weg S in axialer Richtung aufgetragen ist. Für eine Anordnung gemäss Figur 5 erfolgt mit dem zusätzlichen Eingriff von Punkt P₂ ein steilerer Anstieg der Widerstandskraft F. Für eine Anordnung gemäss Figur 7, der eine gestrichelte Kennlinie entspricht, erfolgt mit dem zusätzlichen Eingriff von einem Punkt P₂' zunächst ein Sprung 33 im Kraftanstieg, an den ein steilerer Anstieg anschliesst.

In Figur 9 zeigt eine weitere Anordnung, bei der zunächst die zweite Stufe 11, die mit Rippen 17 versehen ist, mit einem Punkt P₃ in Eingriff mit der konischen Bohrung 5 kommt, während die erste Stufe mit einem Punkt P₄ noch ein Spiel zur konischen Bohrung 5 aufweist. Beim Fortsetzen des Aufsetzens wird in diesem Fall zunächst die Rippe 17 nach innen deformiert, um den Zapfen 7 zu klemmen. Erst wenn der Punkt P₄ an der konischen Bohrung 5 anliegt, wird bei einer Fortsetzung der Bewegung ein Knick mit steilerem Anstieg der Widerstandskraft F erzeugt. Ganz allgemein kann die Grösse der axialen Widerstandskraft durch die Formgebung respektive durch die Kontur, die Durchmesser 10, 12 und den axialen Abstand 14 der beiden Stufen 9, 11, welche ihr Nachgeben in radialer Richtung bestimmen, und durch die Grösse des halben Konuswinkel 8 definiert werden.

## Patentansprüche

1. Einzementierbare Schaftprothese mit einer an ihrem distalen Schaftende (3) aufsteckbaren Zentriervorrichtung (2) aus Kunststoff, welche zum distalen Schaftende (3) einen Hohlraum (4) bildet, wobei zwischen dem distalen Schaftende (3) und der Zentriervorrichtung (2) eine konische Klemmverbindung (6) besteht, die in distaler Richtung ein tieferes Einsinken des distalen Schaftendes (3) in den Hohlraum (4) ermöglicht, **dadurch gekennzeichnet, dass** das distale Schaftende (3) in proximaler Richtung eine konische Bohrung (5) aufweist, in der ein im Hohlraum (4) an die Zentriervorrichtung (2) angeformter Zapfen (7) die Klemmverbindung (6) bildet.

2. Schaftprothese mit Zentriervorrichtung nach Anspruch 1, **dadurch gekennzeichnet, dass** die Zentriervorrichtung (2) als Spritzteil mit einem Spritzwerkzeug hergestellt ist.

3. Schaftprothese mit Zentriervorrichtung nach einem der Ansprüche 1 oder 2, **dadurch gekennzeichnet, dass** die konische Bohrung(5) einen halben Konuswinkel (8) zwischen 0,5° und 2,5° aufweist.

4. Schaftprothese mit Zentriervorrichtung nach Anspruch 3, **dadurch gekennzeichnet, dass** die konische Bohrung (5) einen halben Konuswinkel (8) zwischen 1,2° und 1,6° aufweist.

5. Schaftprothese mit Zentriervorrichtung nach einem der Ansprüche 1 bis 4, **dadurch gekennzeichnet, dass** der Zapfen (7) eine erste Stufe (9) aufweist, an die eine zweite Stufe (11) mit grösserem Durchmesser (12) anschliesst, um am Zapfen (7) zwei Klemm- und Führungszonen (13, 15) mit axialem Abstand (14) zu erzeugen.

6. Schaftprothese mit Zentriervorrichtung nach Anspruch 5, **dadurch gekennzeichnet, dass** die erste Stufe (9) aus mehreren vorstehenden Rippen (16, 17, 18) gebildet ist, die gegen ihre radiale Deformation weniger Widerstand als die zweite Stufe (11) entgegensetzen.

7. Schaftprothese mit Zentriervorrichtung nach einem der Ansprüche 1 bis 6, **dadurch gekennzeichnet, dass** die mögliche Einsinktiefe des Schaftes (3) nach dem Aufstecken und Klemmen der Zentriervorrichtung (2) mehr als 1 mm beträgt.

8. Schaftprothese mit Zentriervorrichtung nach einem der Ansprüche 1 bis 7, **dadurch gekennzeichnet, dass** die Zentriervorrichtung mindestens drei Zentrierflossen (22, 23, 24) aufweist, um den Schaft (3) beim Einsetzen in eine Markraumhöhle (25) an deren Wänden ausrichtbar zu machen.

9. Schaftprothese mit Zentriervorrichtung nach Anspruch 8 für einen Femurschaft, **dadurch gekennzeichnet, dass** eine Flosse (24) gegen lateral ausrichtbar ist, indem innerhalb des Hohlraumes (4) zusätzlich eine formschlüssige Drehsicherung (27, 28) zwischen Schaftende (3) und Zentriervorrichtung (2) im Eingriff ist.

10. Schaftprothese mit Zentriervorrichtung nach einem der Ansprüche 1 bis 9, **dadurch gekennzeichnet, dass** die Zentriervorrichtung aus PMMA besteht.

## Claims

1. Cementable shaft prosthesis including a centering apparatus (2) of plastic which can be pushed onto the distal shaft end (3) of the prosthesis and which forms a cavity (4) to the distal shaft end (3), with a conical clamping connection (6) existing between the distal shaft end (3) and the centering apparatus (2) which permits a deeper sinking of the distal shaft end (3) into the cavity (4) in the distal direction, **characterized in that** the distal shaft end (3) has a conical bore (5) in the proximal direction in which a spigot (7) formed onto the centering apparatus (2) in the cavity (4) forms the clamping connection (6).

2. Shaft prosthesis including a centering apparatus in accordance with claim 1, **characterized in that** the centering apparatus (2) is manufactured as an injection molded part using an injection molding tool.

3. Shaft prosthesis including a centering apparatus in accordance with any one of the claims 1 or 2, **characterized in that** the conical bore (5) has a half cone angle (8) between 0.5° and 2.5°.

4. Shaft prosthesis including a centering apparatus in accordance with claim 3, **characterized in that** the conical bore (5) has a half cone angle (8) between 1.2° and 1.6°.

5. Shaft prosthesis including a centering apparatus in accordance with any one of the claims 1 to 4, **characterized in that** the spigot (7) has a first stage (9) at which a second stage (11) with a larger diameter (12) adjoins in order to produce two clamping and guiding zones (13, 15) with an axial spacing (14) at the spigot (7).

6. Shaft prosthesis including a centering apparatus in accordance with claim 5, **characterized in that** the first stage (9) is formed of a plurality of projecting ribs (16, 17, 18) which provide a lower resistance to their radial deformation than the second stage (11).

7. Shaft prosthesis including a centering apparatus in accordance with any one of the claims 1 - 6, **characterized in that** the possible sinking depth of the shaft (3) amounts to more than 1 mm after the pushing on and clamping of the centering apparatus (2).

8. Shaft prosthesis including a centering apparatus in accordance with any one of the claims 1 - 7, **characterized in that** the centering apparatus has at least three centering fins (22, 23, 24) in order to make the shaft (3) alignable with the walls of the medullary cavity (25) during insertion into the medullary cavity (25).

9. Shaft prosthesis including a centering apparatus in accordance with claim 8 for a femur shaft, **characterized in that** one fin (24) can be oriented towards the lateral **in that** a shape matched security (27, 28) against rotation is additionally in engagement within the cavity (4)between the shaft end (3) and the centering apparatus (2).

10. Shaft prosthesis including a centering apparatus in accordance with any one of the claims 1 to 9, **characterized in that** the centering apparatus consists of PMMA.

## Revendications

1. Prothèse à tige à implanter par cimentation, comportant un dispositif de centrage (2) en matière plastique enfichable sur l'extrémité distale (3) de sa tige et formant une cavité (4) vers l'extrémité distale (3) de la tige, une liaison de serrage conique (6) existant entre l'extrémité distale (3) de la tige et le dispositif de centrage (2), liaison qui permet en direction distale un enfoncement plus profond de l'extrémité distale (3) de la tige dans la cavité (4), **caractérisée en ce que** l'extrémité distale (3) de la tige présente en direction proximale un perçage conique (5) dans lequel un tenon (7) conformé sur le dispositif de centrage (2) dans la cavité (4) établit la liaison de serrage (6).

2. Prothèse à tige comportant un dispositif de centrage selon la revendication 1, **caractérisée en ce que** le dispositif de centrage (2) est réalisé sous forme de pièce injectée au moyen d'un outil d'injection.

3. Prothèse à tige comportant un dispositif de centrage selon l'une ou l'autre des revendications 1 et 2, **caractérisée en ce que** le perçage conique (5) présente un demi-angle de cône (8) compris entre 0,5° et 2,5°.

4. Prothèse à tige comportant un dispositif de centrage selon la revendication 3, **caractérisée en ce que** le perçage conique (5) présente un demi-angle de cône (8) compris entre 1,2° et 1,6°.

5. Prothèse à tige comportant un dispositif de centrage selon l'une des revendications 1 à 4, **caractérisée en ce que** le tenon (7) présente un premier gradin (9) auquel se raccorde un deuxième gradin (11) de plus grand diamètre (12), afin de réaliser sur le tenon (7) deux zones de serrage et de guidage (13, 15) à distance axiale (14).

6. Prothèse à tige comportant un dispositif de centrage selon la revendication 5, **caractérisée en ce que** le premier gradin (9) est formé par plusieurs nervures en saillie (16, 17, 18) qui opposent moins de résistance à leur déformation radiale que le deuxième gradin (11).

7. Prothèse à tige comportant un dispositif de centrage selon l'une des revendications 1 à 6, **caractérisée en ce que** la profondeur possible d'enfoncement de la tige (3) après enfichage et serrage du dispositif de centrage (2) est de plus de 1 mm.

8. Prothèse à tige comportant un dispositif de centrage selon l'une des revendications 1 à 7, **caractérisée en ce que** le dispositif de centrage comprend au moins trois ailes de centrage (22, 23, 24) pour que la tige (3), lors de la mise en place dans une cavité médullaire (25), puisse être orientée au niveau de ses parois.

9. Prothèse à tige comportant un dispositif de centrage selon la revendication 8 pour une tige de fémur, **caractérisée en ce qu'**une aile (24) est orientable latéralement du fait qu'un blocage anti-rotation (27, 28) en coopération de formes est en supplément en engagement entre l'extrémité (3) de la tige et le dispositif de centrage (2) à l'intérieur de la cavité (4).

10. Prothèse à tige comportant un dispositif de centrage selon l'une des revendications 1 à 9, **caractérisée en ce que** le dispositif de centrage est constitué en polyméthyle méthacrylate (PMMA).
